# EUROPEAN PATENT APPLICATION

(11) **EP 1 346 724 A1**
(43) Date of publication of application: **24.09.2003**
(21) Application number: 01271879.7
(22) Date of filing: 25.12.2001
(51) Int. Cl.: A61K 31/7072, A61K 31/7068, A61K 31/706, A61P 31/14, A61P 1/16, C07H 19/073, C07H 19/10, C07H 19/12

(54) **REMEDIES FOR HEPATITIS C**

(30) Priority: 26.12.2000 JP 2000394620; 31.01.2001 JP 2001023542; 04.04.2001 JP 2001105585
(71) Applicant: Mitsubishi Pharma Corporation, Osaka-shi, Osaka 541-0046 (JP)
(72) Inventor: MORIOKA,M. MITSUBISHI PHARMA CORP., Tokyo Head Off, Chuo-ku, Tokyo 1038405 (JP); UBASAWA,M. MITSUBISHI PHARMA CORP., Tokyo Head Off, Chuo-ku, Tokyo 103-8405 (JP); ARAI,M. MITSUBISHI PHARMA CORP., Tokyo Head Office, Chuo-ku, Tokyo 103-8405 (JP)
(74) Representative: von Kreisler, Alek, Dipl.-Chem.
(86) International application number: JP0111365
(87) International publication number: WO02051425

(57) **Abstract**

Excellent remedies for hepatitis C which contain as the active ingredients a 3'-deoxy-3'-fluorouridine derivatives and a 1-(3'-deoxy-fluoro-β-L-ribofuranosyl)uracil derivative and show little side effects.

## Description

### FIELD OF THE INVENTION

The present invention relates to therapeutic agents for hepatitis C, and more particularly, relates to therapeutic agents for hepatitis C including a 3'-deoxy-3'-fluorouridine derivative and a
1-(3'-deoxy-3'-fluoro-β-L-ribofuranosyl)uracil derivative having a specified structure as active ingredients.

### BACKGROUND OF THE INVENTION

Since 1989 when the hepatitis C virus (HCV) was discovered by Chiron Corporation, U.S., understanding of chronic liver diseases have drastically changed. Many liver diseases which have been recognized as non-A, non-Boralcohol-induced hepatitis so far were revealed to be hepatitis C which is caused by an HCV infection. Accordingly, substantial entities thereof have been elucidated one after another. In addition, it has also beenclarified that hepatitis Chardly exhibits serious symptoms, however on the contrary, the majority are chronic and steadily progress, and hepatic cancer develops after a long duration at a high rate.

As clinical achievements, screening upon blood transfusion with an HCV antibody was immediately initiated, which drastically decreased posttransfusion hepatitis. Moreover, the efficacy of interferon has been indicated, and thus medical treatment has been partly expected. However, the efficacy is not more than about 30%, and side effects such as severe depression symptoms etc., have been reported. Recently, efficacy has been indicated through use in combination with ribavirin which is an anti-viral agent, however, it is not recognized to be a sufficiently satisfactory therapeutic method.

Additionally, HCV is a flavivirus having (+)-chain single stranded RNA in its genome, which proliferates by RNA dependent RNA polymerase. It is believed that the proliferation of HCV can be suppressed by specifically inhibiting this RNA dependent RNA polymerase of HCV without inhibiting human RNA polymerase, which may pave the way for effective therapeutic methods of hepatitis C. However, such an effective inhibitor has not been found yet.

On the other hand, 3'-deoxy-3'-fluorouridine was first reported of its synthesis in 1975 by G. Kowolliketal. (G. Kowollik etal., J. Carbohydrate, Nucleosides, Nucleotides, 2(3) 191-195 (1975). Thereafter, a variety of synthetic methods have been reported (Hemant K. Misra et al., J. Heterocyclic Chem. 21, 773 (1984); Japanese Unexamined Patent Publication No. Sho-62-81397; L. Alder et al., Biochemical and Environmental Mass Spectrometry, Vol. 13, 217-221 (1986); A. Van Aerschot et al., Antiviral Research, 12, 133 (1989); Igor A. Mikhailopulo, et al., FEBS. Vol. 250, No. 2, 139 (1989); and J. Med. Chem. 34, 2195 (1991)).

Further, a variety of reports have also been made with respect to pharmacological actions of this compound. A. Van Aerschot et al., Antiviral Research, 12, 133 (1989); Igor A. Mikhailopulo, et al., FEBS. Vol. 250, No. 2, 139 (1989); and J. Med. Chem. 34, 2195 (1991) also disclose that 3' -deoxy-3' -fluorouridine that is one of the active ingredients of the therapeutic agent according to the present invention exhibits antiviral activity, however, it has only a low inhibitory activity on proximate viruses of the hepatitis C virus (i.e., viruses having (+)-chain single stranded RNA in their genome). Thus, an anti-HCV activity has not been suggested.

Moreover, in connection with 3'-deoxy-5,3'-difluorouridine that is one of the active ingredients of the therapeutic agent according to the present invention, Japanese Unexamined Patent Publication No.Sho-62-81397 and Japanese Unexamined Patent Publication No. Sho-62-242624 disclose its synthetic method, however, these do not provide any information from which an anti-HCV activity is expected.

### DISCLOSURE OF THE INVENTION

The present inventors have carried out a thorough investigation in order to provide excellent therapeutic agents for hepatitis C with little side effects, and consequently found that 3'-deoxy-3'-fluorouridine derivatives and 1-(3'-deoxy-3'-fluoro-β-L-ribofuranosyl)uracil derivatives have an enzyme inhibitory activity which is selective to RNA dependent RNA polymerase of HCV, without inhibiting human RNA polymerase. The present invention was thus accomplished.

Accordingly, the present invention provides a therapeutic agent for hepatitis C which comprises a compound selected from a 3'-deoxy-3'-fluorouridine derivative represented by the following general formula (I): 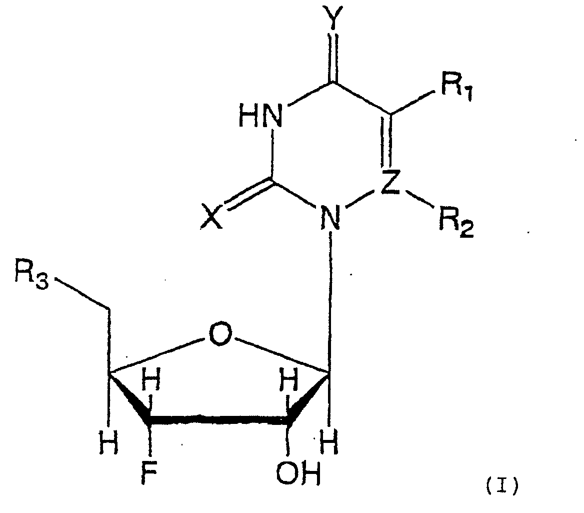 wherein R₁ represents a hydrogen atom, a halogen atom, an alkyl group, an alkyl group substituted by halogen atoms, a hydroxyalkyl group, an aminoalkyl group, an amino group, a formyl group, a cyano group or an unsubstituted or modified benzyl group. Z represents a carbon atom or a nitrogen atom, and when Z represents a carbon atom, R₂ represents a hydrogen atom, a halogen atom, a formyl group or a cyano group. R₃ represents a hydroxyl group or the following formula: 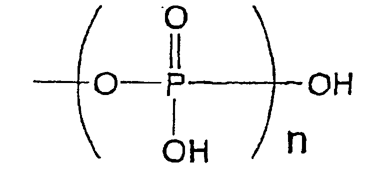 (wherein n represents 1, 2 or 3). X and Y each independently represent an oxygen atom or a sulfur atom,
salts thereof and hydrates or solvates of the same as an active ingredient;
a compound selected from a 1-(3'-deoxy-3'-fluoro-β-L-ribofuranocyl)uracil derivative represented by the following formula (II): 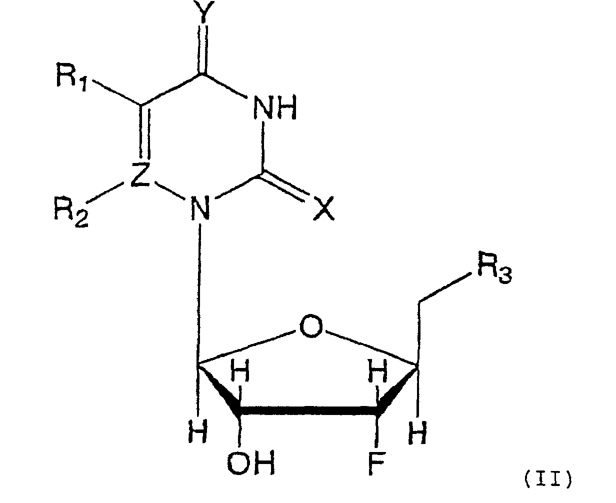 wherein R₁ represents a hydrogen atom, a halogen atom, an alkyl group, an alkyl group substituted by halogen atoms, a hydroxyalkyl group, an aminoalkyl group, an amino group, a formyl group, a cyano group or an unsubstituted or modified benzyl group. Z represents a carbon atom or a nitrogen atom, and when Z represents a carbon atom, R₂ represents a hydrogen atom, a halogen atom, a formyl group or a cyano group. R₃ represents a hydroxyl group or the following formula: 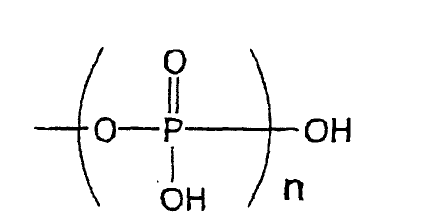 (wherein n represents 1, 2 or 3). X and Y represent an oxygen atom or a sulfur atom,
salts thereof and hydrates or solvates of the same; and
a therapeutic agent for hepatitis C which comprises the compound as an active ingredient.

### BRIEF DESCRIPTION OF THE DRAWINGS

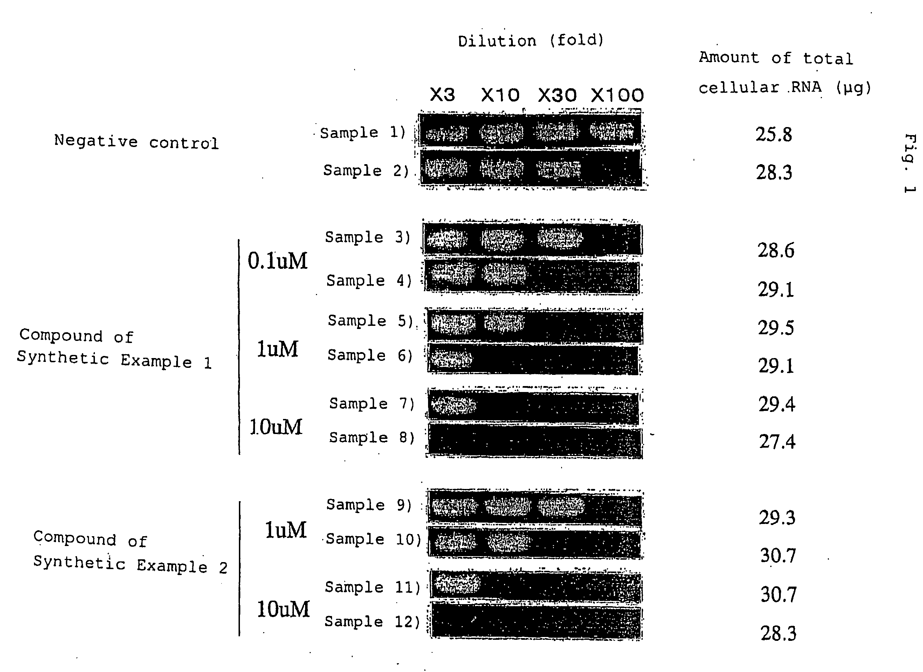

Figure 1 shows photographs of electrophoresis illustrating "detection limit dilution (fold) of an HCV genome" for samples to which the compound of Synthetic Example 1 was added, samples to which the compound of Synthetic Example 2 was added, and samples to which the compound of the present invention was not added; and "total amount of cellular RNA (µg)".

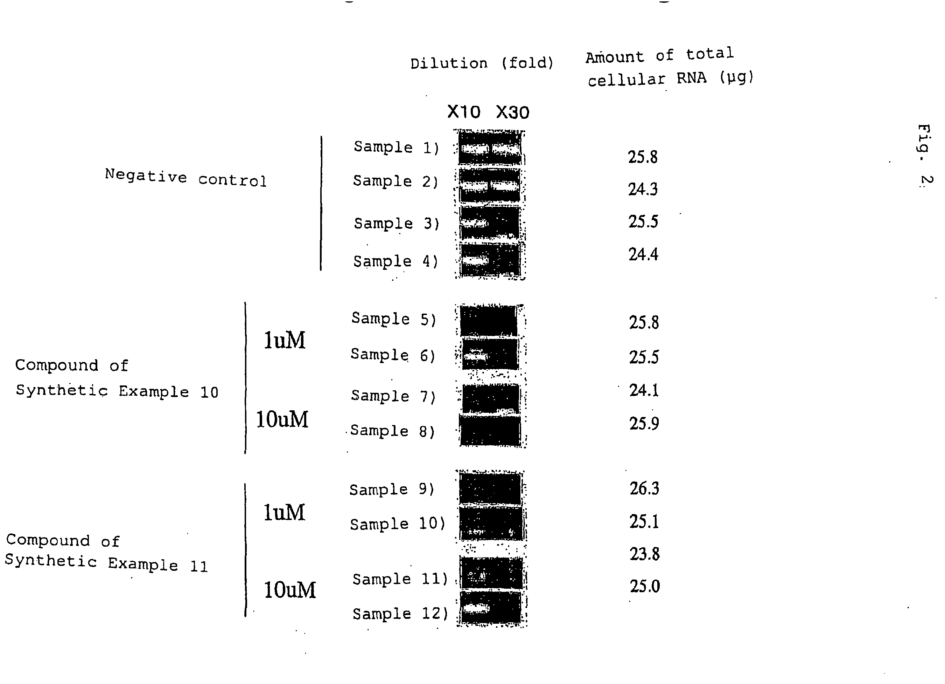

Figure 2 shows photographs of electrophoresis illustrating "detection limit dilution (fold) of an HCV genome" for samples to which the compound of Synthetic Example 10 was added, samples to which the compound of Synthetic Example 11 was added, and samples to which the compound of the present invention was not added; and "total amount of cellular RNA (µg)".

### BEST EMBODIMENT FOR CARRYING OUT THE INVENTION

The compound according to the present invention may include compounds represented by the following general formula (I) and the following general formula (II).

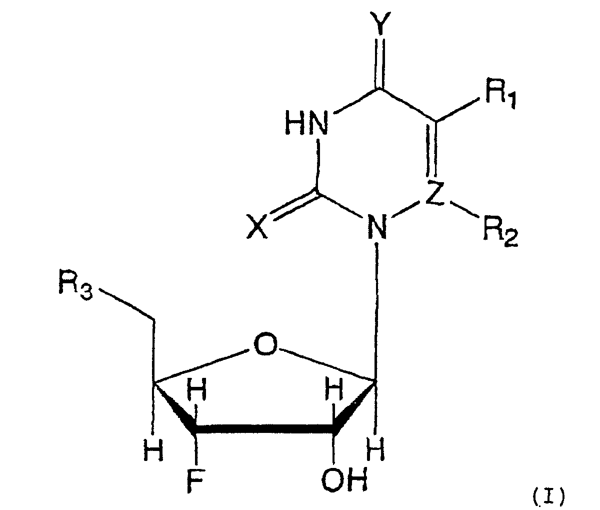

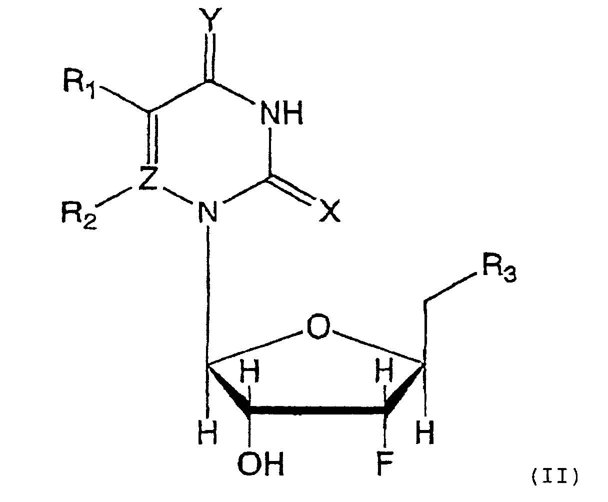

In the above general formula (I) and the above general formula (II), R₁ represents a hydrogen atom, a halogen atom, an alkyl group, an alkyl group substituted by halogen atoms, a hydroxyalkyl group, an aminoalkyl group, an amino group, a formyl group, a cyano group or an unsubstituted or modified benzyl group. Z represents a carbon atom or a nitrogen atom, and when Z represents a carbon atom, R₂ represents a hydrogen atom, a halogen atom, a formyl group or a cyano group. In the definitions of substituents of R₁ and R₂, examples of the halogen atom include a fluorine atom, a chlorine atom, a bromine atom or an iodine atom or the like; examples of the alkyl group include an alkyl group having 1 to 6 carbon atoms, and preferably an alkyl group having 1 to 3 carbon atoms such as a methyl group, an ethyl group, a normal-propyl group, an isopropyl group or the like; examples of the aminoalkyl group include a 4-aminobutyl group, a 3-aminopropyl group, a 2-aminoethyl group, an aminomethyl group or the like. Furthermore, examples of the group to modify the benzyl group include a halogen atom, an alkyl group, an alkyl group substituted by halogen atoms, a hydroxyalkyl group, an aminoalkyl group, an amino group, a formyl group, a cyano group and the like. Specific examples of these are as described above.

R₃ represents a hydroxyl group or the following formula: 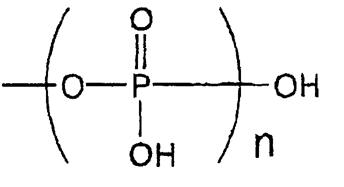 (wherein n represents 1, 2 or 3). X and Y each independently represent an oxygen atom or a sulfur atom.

Specific examples of the 3'-deoxy-3'-fluorouridine derivative represented by the above general formula (I) and the 1-(3'-deoxy-3'-fluoro-β-L-ribofuranosyl)uracil derivative represented by the above general formula (II) include the compounds below.

The 3'-deoxy-3'-fluorouridine derivative represented by the above general formula (I) include
3'-deoxy-3'-fluorouridine,
3'-deoxy-5,3'-difluorouridine,
3'-deoxy-3'-fluoro-5-methyluridine,
3'-deoxy-3'-fluoro-5-cyanouridine,
3'-deoxy-3'-fluoro-5-trifloromethyluridine,
3'-deoxy-3'-fluoro-5-bromouridine,
3'-deoxy-3'-fluoro-6-azauridine,
3'-deoxy-3'-fluoro-2-thiouridine,
3'-deoxy-3'-fluoro-4-thiouridine,
3'-deoxy-3'-fluorouridine 5'-triphosphate,
3'-deoxy-5,3'-difluorouridine 5'-triphosphate,
3'-deoxy-3'-fluoro-5-methyluridine 5'-triphosphate,
3'-deoxy-3'-fluoro-5-cyanouridine 5'-triphosphate,
3'-deoxy-3'-fluoro-5-trifloromethyluridine 5'-triphosphate,
3'-deoxy-3'-fluoro-5-bromouridine 5'-triphosphate,
3'-deoxy-3'-fluoro-6-azauridine 5'-triphosphate,
3'-deoxy-3'-fluoro-2-thiouridine 5'-triphosphate,
3'-deoxy-3'-fluoro-4-thiouridine 5'-triphosphate.

Preferable compounds include
3'-deoxy-3'-fluorouridine,
3'-deoxy-5,3'-difluorouridine,
3'-deoxy-3'-fluoro-5-methyluridine,
3'-deoxy-3'-fluorouridine 5'-triphosphate,
3'-deoxy-5,3'-difluorouridine 5'-triphosphate,
3'-deoxy-3'-fluoro-5-methyluridine 5'-triphosphate.

Particularly preferable compounds include
3'-deoxy-3'-fluorouridine,
3'-deoxy-5,3'-difluorouridine,
3'-deoxy-3'-fluoro-5- methyluridine.

The most preferable compounds include
3'-deoxy-3'-fluorouridine.

The 1-(3'-deoxy-3'-fluoro-β-L-ribofuranosyl)uracil derivative represented by the above general formula (II) include
1-(3'-deoxy-3'-fluoro-β-L-ribofuranosyl)uracil,
1-(3'-deoxy-5,3'-difluoro-β-L-ribofuranosyl)uracil,
1-(3'-deoxy-3'-fluoro-5-methyl-β-L-ribofuranosyl)uracyl,
1-(3'-deoxy-3'-fluoro-β-L-ribofuranosyl)uracil 5'-triphosphate,
1-(3'-deoxy-5,3'-difluoro-β-L-ribofuranosyl)uracil 5'-triphosphate,
1-(3'-deoxy-3'-fluoro-5-methyl-β-L-ribofuranosyl)uracil 5'-triphosphate.

Preferable compounds include
1-(3'-deoxy-3'-fluoro-β-L-ribofuranosyl)uracil,
1-(3'-deoxy-5,3'-difluoro-β-L-ribofuranosyl)uracil,
1-(3'-deoxy-3'-fluoro-5-methyl-β-L-ribofuranosyl)uracil.

Particularly preferable compounds include
1-(3'-deoxy-3'-fluoro-β-L-ribofuranosyl)uracil.

Among the 3'-deoxy-3'-fluorouridine derivatives represented by the above general formula (I) which may be an active ingredient according to the present invention, 3'-deoxy-3'-fluorouridine, 3'-deoxy-5,3'-difluorouridine and 3'-deoxy-3'-fluoro-5-methyluridine are known compounds which are described in Hemant K. Misra et al., J. Heterocyclic Chem., 21, 773 (1984); Noyori et al., Japanese Unexamined Patent Publication No. Sho-62-81397; L. Alder et al., Biochemical and Environmental Mass Spectrometry, Vol. 13, 217-221 (1986); A. Van Aerschot et al., Antiviral Research, 12, 133 (1989); Igor A. Mikhailopulo, et al., J. Med. Chem. 34, 2195 (1991), and thus they can be produced according to the disclosure therein. Also, the 3'-deoxy-3'-fluorouridine derivatives represented by the above general formula (I) other than these compounds can be similarly produced on the basis of the disclosure of the same. In addition, the 1-(3'-deoxy-3'-fluoro-β-L-ribofuranosyl)uracil derivatives represented by the above general formula (II) are L-form enantiomers of the above known compounds in the D-form. Thus they can also be produced pursuant to the disclosure of these documents by changing the sugar raw material into L-xylose.

The 3'-deoxy-3'-fluorouridine derivative represented by the above general formula (I) and the 1- (3'-deoxy-3'-fluoro-β-L-ribofuranosyl)uracil derivative represented by the above general formula (II) can be formed into physiologically acceptable salts. Examples of the salt include alkaline metal salts, alkaline earth metal salts, ammonium salts, alkylammonium salts or the like.

Further, the 3'-deoxy-3'-fluorouridine derivative represented by the above general formula (I) and the 1-(3'-deoxy-3'-fluoro-β-L-ribofuranosyl)uracil derivative represented by the above general formula (II) and salts thereof can be optionally formed into hydrates or solvates of the same. Examples of the solvate include methanol, ethanol, isopropanol, acetone, ethyl acetate, methylene chloride and the like.

The aforementioned salts and hydrates or solvate of the same are also included in the scope of the active ingredient according to the present invention.

The 3' -deoxy-3' -fluorouridine derivatives represented by the above general formula (I) and the 1-(3'-deoxy-3'-fluoro-β-L-ribofuranosyl)uracil derivatives represented by the above general formula (II) are compounds having an antiviral activity, anti-RNA virus activity, anti-hepatitis C virus activity, and thus they can be used as a therapeutic agent for hepatitis C.

Although the above-described active ingredient itself may be used in its unmodified form as the therapeutic agent for hepatitis C of the present invention, to use a pharmaceutical composition comprising the above-described active ingredient is preferred which is produced through using generalized additives for formulations. Examples of dosage form of the pharmaceutical composition include tablets, capsules, subtle granules, pills, lozenges, liquids, injections, suppositories, ointments, patches or the like. These can be orally (including sublingual administration) or parenterally administered.

Pharmaceutical compositions for use in oral administration can be produced by any conventional frequently-used method such as mixing, filling or tablet making or the like. Furthermore, the active ingredient maybe distributed within a pharmaceutical composition in which a large amount of filler is used through employing a repeated blending manipulation.

For example, it is preferable that a tablet or a capsule which is used for oral administration is provided as a unit dosage form, which may contain a typically employed carrier for a formulation such as a binder, a filler, a diluent, an agent for tablet making, a lubricant, a disintegrant, a colorant, a flavoring agent, a wetting agent and the like. The tablet may be formed to give a coating tablet according to any known method in the art, for example, using a coating agent.

Examples of preferable filler include cellulose, mannitol, lactose or the like. Moreover, as further additives for a formulation, a disintegrant such as starch derivatives e.g., starch, polyvinylpyrrolidone, sodium starch glycolate or the like, lubricants such as sodium lauryl sulfate or the like.

Examples of the pharmaceutical composition in the form of a liquid formulation for use in oral administration include water based or oil based suspensions, solutions, emulsions, syrups, elixirs, dry pharmaceutical compositions which can be redissolved in water or a suitable medium prior to use, or the like.

Such a liquid formulation may include usual additives, for example, a suspending agent such as sorbitol, a syrup, methylose, an aluminum stearate gel, hydrogenated edible fat or the like; an emulsifying agent such as lecithin, sorbitan monooleate, gum arabic or the like; an oily ester such as almond oil, rectified coconut oil, a glycerin ester or the like; non water based medium such as propylene glycol, ethyl alcohol or the like (may also involve edible oil); a preservative such as methyl ester, ethyl ester or propyl ester of p-hydroxybenzoic acid, sorbic acid or the like; and furthermore, general flavoring agents or colorants, or the like as needed.

Examples of the pharmaceutical composition which are suitable for parenteral administration include pharmaceutical compositions in liquid form, suppositories, patches andthe like, which comprise the active ingredient as described above and a sterilized medium. For example, the active ingredient can be suspended, dissolved or emulsified depending on the medium or the concentration, and for example, the composition in a liquid form for use in parenteral administration can be produced preferably by dissolving the active ingredient in a medium followed by sterilization and filtration, and then filling in an appropriate vial or ampule and sealing. In order to improve stability, moisture may be eliminated by freeze-drying after preparing the composition in an aqueous solution form.

The suspension for use in parenteral administration can be produced by a method which is substantially similar to the method for composition in a solution form for use in parenteral administration. For example, it can be produced by suspending the active ingredient in a medium, sterilizing with ethylene oxide or the like, followed by additional suspension in a sterilized medium. Upon production of the suspension or the like, a surfactant or a wetting agent may be added as needed so that the active ingredient is uniformly distributed in the formulation. Pharmaceutical compositions in another form can also be produced by any method known to persons skilled in the art. Accordingly, the form and the process for producing the pharmaceutical composition in one embodiment of the medicine of the present invention are not limited to those as described above.

The pharmaceutical composition for use in oral administration (for example, in cases of tablets, capsules, subtle granules and the like) generally includes 5 to 95% by weight, and preferably 25 to 90% by weight of the active ingredient. The pharmaceutical composition for use in parenteral administration (for example, in cases of injections) generally includes 0.5 to 20% by weight, and preferably 1 to 10% by weight of the active ingredient.

The therapeutic agent of the present invention is useful in the medical treatment of hepatitis caused by HCV. The dose of the therapeutic agent of the present invention may be determined adequately depending on age, health conditions, body weight, severity of disease of the patient, type and frequency of the medical treatment or procedure which is concurrently performed, properties of the desired effect and the like. In general, the dosage for an adult per dose shall be 0.01 to 50 mg/kg body weight, preferably 1 to 30 mg/kg body weight as an amount of the active ingredient, and administered once to several times per day.

### EXAMPLES

The present invention is more specifically explained below with reference to Synthetic Examples and Examples, however, the following Synthetic Example s and Examples should be construed as merely an aid in specific understanding in connection with the present invention. Accordingly, the scope of the present invention is not in any way limited by the following Synthetic Example s and Examples.

### <Synthetic Example 1> Synthesis of 3'-deoxy-3'-fluorouridine

3'-Deoxy-3'-fluorouridine used in the following Example 2 was produced according to the Synthetic Example in J. Med. Chem. 34, 2195 (1991).
¹H-NMR(DMSO-d6): δ 3.5-3.7(m,2H,H-5') 4.05-4.25(m,1H,H-4') 4.1-4.3(m,1H,H-2') 4.93(dd,j=4.2Hz,54.9Rz,1H,H-3') 5.26(t,J=5.1Hz, 1H, 5' 0H) 5.70(d,J=8.1Hz, 1H, H-5) 5.83(d,J=6.3Hz, 1H,2' 0H) 5.87(d,J=7. 8Hz, 1H, H-1') 7.81(d,1H,H-6) 11.35(br-s,NH)

### <Synthetic Example 2> Synthesis of 3'-deoxy-5,3'-difluorouridine

3'-Deoxy-5,3'-difluorouridine used in the following Example 2 was produced according to the Synthetic Example in J. Med. Chem. 34, 2195 (1991).

To an acetonitrile solution (0.6mL)of 5-fluorouracil (29 mg) was added bistrimethylsilyl acetamide (135 µL) followed by stirring for 1 hour. After the reaction mixture became transparent, thereto was added a solution of 1-O-acetyl-2,5-di-O-benzoyl-3-deoxy-3-fluoro-α,β-D-ribofura nosyl (89 mg) in acetonitrile (1 mL). Under ice-cooling of the reaction mixture, thereto was added tin (IV) chloride (34 µL) followed by heating under reflux for 8 hours. The reaction mixture was poured into cold water, extracted with ethyl acetate, dried over magnesium sulfate, followed by filtration and concentration to yield 80 mg of residue. This residue was dissolved in 1 M ammonia in methanol, followed by stirring at room temperature for 120 hours. The reaction mixture was concentrated and washed with diethyl ether to yield the title compound (21 mg).
¹H-NMR(DMSO-d₆) : δ 3.5-3.7(m,2H,H-5') 4.05-4.25(m,1H,H-4') 4.1-4.3(m,1H,H-2') 4.94(dd,j=4.1Rz,54.5Hz,1H,R-3') 5.40(br-s, 1H, 2' 0H) 5.85(d,J=7.5Hz,1R,H-1') 7.94(d,J=8.0Rz,1H,H-6)

### <Synthetic Example 3> Synthesis of 3'-deoxy-3'-fluoro-5-methyluridine

Similar to Synthetic Example 2 except that 5-methyluracil was used instead of 5-florouracil, the title compound was obtained.
¹H-NMR 270MHz(DMSO- d₆): δ ppml.77(s,3H,CH₃) 3.50-3.70(m,2H,H-5') 4.05-4.25(m,1H.H-4') 4.10-4.30(m,1H.H-2') 4.93(dd,J=4.3Hz, 54.8Hz,1H,H-3') 5.28(t,J=5.1Hz,1H,5' 0H) 5.79(d,J=6.3Hz,1H,2' 0H) 5.87(d,J--8. 1Hz, 1H,H-1') 7.67(s,1H,H-6) 11.37(br-s,1H,NH)

### <Synthetic Example 4> Synthesis of 3'-deoxy-3'-fluoro-5-cyanouridine

Similar to Synthetic Example 2 except that 5-cyanouracil was used instead of 5-florouracil, the title compound was obtained.
¹H-NMR 270MHz(DMSO-d₆): δ ppm3.50-3.70(m,2H,H-5') 4.05-4.25(m,1H,H-4') 4.10-4.30(m,1H,H-2') 4.99(d,J=56.1Hz,1H,H-3') 5.49(t,J=4.4Hz,1H,5'0H) 5.84(d,J=6.4Rz,1R,1'-H) 5.91(d,J=5.4Hz,1H,2'H) 8.78(s,1H,H-6) 12.15(br-s,1H,NH). IR: 2238cm-¹ (C=N)

### <Synthetic Example 5 > Synthesis of 3'-deoxy-3'-fluoro-5-trifloromethyluridine

Similar to Synthetic Example 2 except that 5-trifloromethyluracil was used instead of 5-florouracil, the title compound was obtained.
¹H-NMR 270MHz(DMSO-d₆): δ ppm3.50-3.70(m,2H,H-5') 4.05-4.25(m,1H,H-4') 4.10-4.30(m,1H,H-2') 4.99(dd,J=2.8Hz,53.8Hz,1H,H-3') 5.50(br-s,1H,5'0H) 5.84(br-s,1H,2'0H) 5.90(d,J=6.8Hz,1H,1'-R) 8.68(s,1H,H-6) 10.95(br-s,1H,NH)

### <Synthetic Example 6> Synthesis of 3'-deoxy-3'-fluoro-5-bromouridine

Similar to Synthetic Example 2 except that 5-bromouracil was used instead of 5-florouracil, the title compound was obtained.
¹H-NMR 270MHz(DMSO-d₆): δ ppm3.50-3.70(m,2H,H-5') 4.10-4.25(m,1H,H-4') 4.10-4.30(m,1H,H-2') 4.96(dd,J=3.6Hz,54.1Hz,1H,H-3') 5.42(br-s,1H,5'0H) 5.86(br-s, 1H,2' 0H) 5.88(d,J=6. 8Hz,1H, 1'-H) 8.34(s,1H,H-6)

### <Synthetic Example 7> Synthesis of 3'-deoxy-3'-fluoro-6-azauridine

Similar to Synthetic Example 2 except that 6-azamouracil was used instead of 5-florouracil, the title compound was obtained.
¹H-NMR 270MHz(DMSO-d₆): δ ppm3.28-3.50(m,2H,H-5') 4.00-4.16(m,1R,R-4') 4.40-4.62(m,1H,H-2') 4.89(t,J=5.1Hz,1H,5'0H) 5.00(dd,J=4.3Hz,54.1Hz,1H,H-3') 5.77(d,J=6.1Hz,1H,2'0H) 5.91(d,J=6.8Hz,1H,1'-H) 7.61(s,1H,H-6) 12.33(br-s,1H,NH)

### <Synthetic Example 8> Synthesis of 3'-deoxy-3'-fluoro-2-thiouridine

Similar to Synthetic Example 2 except that 2-thiouracil was used instead of 5-florouracil, the title compound was obtained.
¹H-NMR 270MHz(DMSO-d₆): δ ppm3.50-3.70(m,2H,H-5') 4.15-4.25(m,1H,H-4') 4.20-4.30(m,1H,H-2') 4.96(dd,J=4.5Hz,53.6Hz,1H,H-3') 5.38(t,J=4.9Hz,1H,5'0H) 5.86(d,J=6.1Hz,1H,2'0H) 6.06(d,J=8.1Hz,1H,5-H) 6.89(d,7.3Hz,1H,1'-H) 8.01(d,J=8,1Hz,1H,H-6) 12.69(br-s,1H,NH). UV: Λmax278nm

### <Synthetic Example 9> Synthesis of 3'-deoxy-3'-fluoro-4-thiouridine

Similar to Synthetic Example 2 except that 4-thiouracil was used instead of 5-florouracil, the title compound was obtained.
¹H-NMR 300MHz(DMSO-d₆): δ ppm3.5-3.7(m,2H,H-5') 4.17-4.2+(m,1N,H-4') 4.20-4.30(m,1H,H-2') 5.00(dd,J=4.1Hz,58.5Hz,1H,H-3') 5.31(br,1H,5'0H) 5.86(d,J=6.1Hz,1H,2'0H) 5.89(d,7.7Hz,1H,1'-H) 6.40(d,J=7.5Hz,1H,5-H) 7.75(d,J=7.5Hz,1H,H-6) 12.79(br,1H,NH). UV: Λmax 327nm

### <Synthetic Example 10> Synthesis of 1-(3'-deoxy-3'-fluoro-β-L-ribofuranosyl)uracil

1-(3'-Deoxy-3'-fluoro-β-L-ribofuranosyl)uracil was produced according to the Synthetic Example in Japanese Unexamined Patent Publication No. Sho-62-81397 and J. Med. Chem. 34, 2195 (1991) using L-xylose (Tokyo Kasei) as a raw material.
¹H-NMR(DMSO-d₆): δ ppm3.5-3.7(m,2H,H-5') 4.05-4.25(m,1H,H-4') 4.1-4.3(m,1H,H-2') 4.93(dd,J=4.2Hz,54.9Hz,1H,H-3') 5.26(t,J=5.1Hz,1H,5'0H) 5.70(d,J=8.1Hz,1H,H-5) 5.83(d,J=6.3Hz,1H,2'0H) 5.87(d,J=7.8Hz,1H,H-1') 7.81(d, 1H,H-6) 11.25(br-s,NH)
Specific rotation: [α]₀²⁶=+42.7⁰(C=0.3,DMSO)

### <Synthetic Example 11> Synthesis of 1-(3'-deoxy-5,3'-difluoro-β-L-ribofuranosyl)uracil

1-(3'-Deoxy-5,3'-difluoro-β-L-ribofuranosyl)uracil used in the following Example 2 was produced according to the Synthetic Example in Japanese Unexamined Patent Publication No. Sho-62-81397 and J. Med. Chem. 34, 2195 (1991) using L-xylose (Tokyo Kasei) as a raw material.

To an acetonitrile solution (1.0 mL)of 5-fluorouracil (37 mg) was added bis(trimethylsilyl)acetamide (172 µL) followed by stirring for 1 hour. After the reaction mixture became transparent, thereto was added a solution of 1-O-acetyl-2, 5-di-O-benzoyl-3-deoxy-3-fluoro-α,β-L-ribofuranosyl (113 mg) in acetonitrile (1 mL). Under ice-cooling of the reaction mixture, thereto was added tin chloride (43 µL) followed by heating under reflux for 8 hours. The reaction mixture was poured into cold water, extracted with ethyl acetate, dried over magnesium sulfate, followed by filtration and concentration to yield 159 mg of a residue. The residue was purified on a silica gel chromatography to yield a product of 119 mg. This product was dissolved in 1 M ammonia in methanol, followed by stirring at room temperature for 15 hours. The reaction mixture was concentrated and washed with diethyl ether to yield the title compound (16 mg).
¹H-NMR(DMSO-d₆): δ 3.5-3.7(m,2H,H-5') 4.05-4.25(m,1H,H-4') 4.1-4.3(m,1H,H-2') 4.94(dd,J=4.1Hz,54.5Hz,1H,H-3') 5.40(br-s,1H,2'0H) 5.85(d,J=7.5Hz,1H, H-1^{'}) 7.94(d,J=8.0Hz,1R,H-6)

Furthermore, the uridine derivatives involving the compounds according to Synthetic Example 1 through Synthetic Example 11 are referred to as being triphosphorylated via monophosphate and diphosphate in a living body. Therefore, triphosphates of uridine derivatives and L-uridine derivatives. were produced as follows.

### <Synthetic Example 12> Synthesis of 3'-deoxy-3'-fluorouridine 5'-triphosphate

Synthesis was performed according to the method by D. Broom et al., (J. Chem. Soc., Chem. Commun., 1276-1277 (1991). In 130 µL of triethyl phosphate was dissolved 4 mg of 3'-deoxy-3'-fluorouridine, and thereto were added 22µL of tri n-butylamine and 7.3 µL of phosphorous oxychloride under an ice-cold condition followed by stirring for 1 hour. The reaction was confirmed by ion exchange based HPLC, and the addition of tri n-butylamine and phosphorous oxychloride in an appropriate amount was repeated until the monophosphate was present as a principal ingredient. To this reaction mixture were added a solution of tri n-butylammonium pyrophosphate (260 µmol) in DMF (390 µL) and 78 µL of tri n-butylamine followed by additional stirring for 1 hour under an ice-cold condition. Thereto was added a 0.1 M aqueous triethylammonium bicarbonate solution (2.6 mL) to terminate the reaction. The product was separated on a DEAE cellulose column. After subjecting to freeze-drying, the product was dissolved in water to yield a predetermined concentration.
³¹P-NMR(standard: H2P04, D20) δ -8.96(dd), -21.92(t)

### <Synthetic Example 13> Synthesis of 3'-deoxy-5,3'-difluorouridine 5'-triphosphate

Title compound was synthesized in a similar manner as Synthetic Example 12.
³¹P-NMR(standard: H2P04, D20) δ -10.34(dd), -20.17(t)

### <Synthetic Example 14> Synthesis of 3'-deoxy-3'-fluoro-5-methyluridine 5'-triphosphate

Title compound was synthesized in a similar manner as Synthetic Example 12.
³¹P-NMR(standard: H2P04, D20) δ ppm-9.41(dd), -22.27(t)

### <Synthetic Example 15> Synthesis of 3'-deoxy-3'-fluoro-5-cyanouridine 5'-triphosphate

Title compound was synthesized in a similar manner as Synthetic Example 12.

³¹P-NMR(standard: H2P04, D20) δ ppm-7.74(d), -10.71(d), -21.38(t)

### <Synthetic Example 16> Synthesis of 3'-deoxy-3'-fluoro-5-trifloromethyluridine 5'-triphosphate

Title compound was synthesized in a similar manner as Synthetic Example 12.
³¹P-NMR(standard: H2P04, D20) δ ppm-6.99(d), -11.04(d), -19.98(t)

### <Synthetic Example 17> Synthesis of 3'-deoxy-3'-fluoro-5-bromouridine 5'-triphosphate

Title compound was synthesized in a similar manner as Synthetic Example 12.
³¹P-NMR(standard: H2P04, D20) δ ppm-9.23(d), -10.94(d), -21.95(t)

### <Synthetic Example 18> Synthesis of 3'-deoxy-3'-fluoro-6-azauridine 5'-triphosphate

Title compound was synthesized in a similar manner as Synthetic Example 12.
³¹P-NMR(standard: H2P04, D20) δ ppm-9.71(d), -10.75(d), -22.24(t)

### <Synthetic Example 19> Synthesis of 3'-deoxy-3'-fluoro-2-thiouridine 5'-triphosphate

Title compound was synthesized in a similar manner as Synthetic Example 12.
³¹P-NMR(standard: H2P04, D20) δ ppm-9.44(d), -10.79(d), -21.96(t)

### <Synthetic Example 20> Synthesis of 3'-deoxy-3'-fluoro-4-thiouridine 5'-triphosphate

Title compound was synthesized in a similar manner as Synthetic Example 12.
³¹P-NMR(standard: H2P04, D20) δ ppm-7.91(d), -10.60(d), -21.20(t)

### <Synthetic Example 21> Synthesis of 1-(3'-deoxy-3'-fluoro-β-L-ribofuranosyl)uracil 5'-triphosphate

Title compound was synthesized in a similar manner as Synthetic Example 12.
³¹P-NMR(standard: H2P04, D20) δ -7.46(dd), -21.76(t)

### <Synthetic Example 22> Synthesis of 1-(3'-deoxy-5,3'-fluoro-β-L-ribofuranosyl)uracil 5'-triphosphate

Title compound was synthesized in a similar manner as Synthetic Example 12.
³¹P-NMR(standard: H2P04, D20) δ -9.49(dd), -20.62(t)

### <Example 1> Inhibitory effect on hepatitis C virus (HCV) RNA polymerase activity

Inhibitory effect on an HCV RNA polymerase activity of the compounds of Synthetic Example 12, Synthetic Example 13, Synthetic Example 19, Synthetic Example 21 and Synthetic Example 22 described above was measured by the following process.

Expression and purification of HCV RNA polymerase were performed in accordance with known processes (V. Lohman et al., VIROLOGY, 249, 108-118, 1998).

The purified HCV RNA polymerase in an amount of 200 ng was mixed with 100 ng/mL poly(A)RNA (polyadenine RNA) as a template RNAand 25 ng/mL 15-meroligo (U) RNA (oligouridine having 15 bases, TAKARA Shuzo) as a primer RNA in 50 µL of an aqueous solution containing 20 mM Tris-Cl (pH 7.5), 5 mM MgCl₂ 25 mM KCl, 10 uM UTP (Uridine-triphosphate), 0.25 Ci/mL [α-³²P]UTP (Amersham, PB10203). Then, thereto was added the compound of Synthetic Example 12, Synthetic Example 13, Synthetic Example 19, Synthetic Example 21 or Synthetic Example 22, followed by an RNA synthetic reaction at 30°C for 30 minutes.

After completing the reaction, RNA in the solution was absorbed on DE81 paper (Whattman). Then, [α-³²P]UTP which was not incorporated into the synthesized RNA was washed out with 0.5 M phosphate buffer, and the quantity of [α-³²P]UTP which was incorporated into the synthesized RNA on the DE81 paper was determined on a liquid scintillation counter.

Inhibitory potency of the compounds of Synthetic Example 12, Synthetic Example 13, Synthetic Example 19, Synthetic Example 21 and Synthetic Example 22 was represented by 50% inhibitory concentration when the negative control without HCV RNA polymerase was assumed as 0% while the positive control without the compound was assumed as 100%.

**<Table 1>**

| 50% inhibitory concentration for HCV RNA polymerase | (µM) |
|---|---|
| 3'-deoxy-3'-fluorouridine 5'-triphosphate (compound of Synthetic Example 12) | 0.3 |
| 3'-deoxy-5,3'-difluorouridine 5'-triphosphate (compound of Synthetic Example 13) | 1.0 |
| 3'-deoxy-3'-fluoro-2-thiouridine 5'-triphosphate (compound of Synthetic Example 19) | 3.0 |
| 1-(3'-deoxy-3'-fluoro-β-L-ribofuranosyl)uracil 5'-triphosphate (compound of Synthetic Example 21) | 3.0 |
| 1-(3'-deoxy-5,3'-difluoro-β-L-ribofuranosyl)uracil 5'-triphosphate (compound of Synthetic Example 22) | 10 |

### <Example 2> Inhibitory effect on hepatitis C virus (HCV) proliferation

Inhibitory effect on HCV proliferation of the compounds of Synthetic Example 1, Synthetic Example 2, Synthetic Example 10 and Synthetic Example 11 described above was measured by the following process.

Measurement of anti HCV proliferation inhibitory activity was performed in accordance with a known process (Kato, N. et. al., Biochem. Biophs. Res. Commun. 206, 863-869, 1995).

MT-2C cells that are cells derived from human T cells (Kato, N. et. al., Biochem. Biophs. Res. Commun. 206, 863-869, 1995) were cultured in an RPMI 1640 medium (Gibco-BRL 11875-101) supplemented with FBS (Foetal Bovine Serum,Gibco-BRL10082-147) at 10%. Thus cultured MT-2C cells (1 x 10⁶ cells) were suspended in 200 uL of an F-12 medium (Gibco-BRL 11059-029) added with EDTA (ethylenediamine tetraacetic acid) to yield final concentration of 5 mM. Thereto was added 20 µL of a serum derived from a hepatitis C patient, followed by infection at 37°C for 2 hours. After the infection, the cells were washed three times with 1 mL of PBS (Phosphate buffered saline), suspended in 6 mL of an RPMI medium added with FBS at 10%. The suspended cells were seeded on a 48 well culture plate, and incubated in a CO₂ incubator at 32°C for 3 days.

Thereto was added the compound of Synthetic Example 1, Synthetic Example 2, Synthetic Example 10 or Synthetic Example 11 dissolved in DMSO (Dimethyl Sulfoxide) in an amount of thousandths of the medium, and the mixture was cultured for 3 days. A medium adjusted to have the same concentration of the identical compound was added to each well in a two-fold volume, followed by an additional culture for 4 days. Cells were harvested for each of the samples, and total cellular RNA was purified. For the RNA purification, ISOGEN (NIPPON GENE, 311-02501) was used. In order to quantitatively determine an HCV RNA genome in the purified RNA, the following operation was carried out.

Using 1 µg of the purified RNA, a reverse transcription reaction was performed in a total volume of 20 uL at 37°C for 1 hour. Subsequently, an inactivation treatment of the reverse transcriptase was carried out at 100°C for 30 minutes. Superscript™ II (Gibco BRL 18064-014) was used as a reverse transcriptase, and an oligoDNA (Gibco BRL) having 20 bases which are complementary to the sequence of base position 317 through base position 336 of HCV-J strain (Kato, N. et. al. Proc. Natl. Acad. Sci. USA 87, 9524-9528, 1990) was used as a primer.

Product of the reverse transcription reaction was diluted to 3-fold, 10-fold, 30-fold and 100-fold with sterilized and distilled water. First PCR (Polymerase Chain Reaction), (34 cycles of: at 94°C for 1 minute, at 58°C for 45 seconds and at 72°C for 1 minute; and 1 cycle of: at 94°C for 1 minute, at 58°C for 1 minute and 40 seconds and at 72°C for 8 minutes) was performed in a total volume of 50 µL using 3 µL of the diluted product of the reverse transcription reaction. EX-Taq (TAKARA RR001A) was used as a DNA synthetase, while an oligoDNA (Gibco BRL) having 20 bases which correspond to the sequence of base position 71 through base position 90 and an oligoDNA (Gibco BRL) having 20 bases which are complementary to the sequence of base position 317 through base position 336 of HCV-J strain were used as primers.

Second PCR reaction (34 cycles of: at 94°C for 1 minute, at 58°C for 45 seconds and at 72°C for 1 minute; and 1 cycle of: at 94°C for 1 minute, at 58°C for 1 minute and 40 seconds and at 72°C for 8 minutes) was performed in a total volume of 50 µL using 2 µL of the reaction product of the first PCR. EX-Taq (TAKARA) was used as a DNA synthetase, while an oligoDNA (Gibco BRL) having 20 bases which correspond to the sequence of base position 122 through base position 141 and an oligoDNA (Gibco BRL) having 20 bases which are complementary to the sequence of base position 246 through base position 265 of HCV-J strain were used as primers.

The reaction product of the second PCR in an amount of 5 µL was electrophoresed on a 3% agarose gel, followed by ethidium bromide staining to detect a DNA fragment.

As a result, an HCV genome could be detected in the negative control - samples 1) and 2), without including any compound, up to 30-fold or 100-fold dilution, as shown in Fig. 1. To the contrary, in the case of the samples added with the compound of Synthetic Example 1, the HCV genome could not be detected: in 0.1 µM added -samples 3) and 4) up to 10-fold or 30-fold; in 1.0 µM added -samples 5) and 6) up to 3-fold or 10-fold; and in 10 µM added -samples 7) and 8) up to 3-fold or at 3-fold, also as shown in Fig. 1. Accordingly, it was revealed that detection limit dilution of the HCV genome decreased depending on the concentration of the compound. In addition, also in the case of the samples added with the compound of Synthetic Example 2, the HCV genome could not be detected: in 1.0 µM added -samples 9) and 10) up to 10-fold or 30-fold; and in 10 µM added -samples 11) and 12) up to 3-fold or at 3-fold, revealing that detection limit dilution of the HCV genome decreased depending on the concentration of the compound. On this occasion, yield of total RNA for each of the samples showed little or no alteration. Thus, it was believed that these compounds inhibited the proliferation of HCV within cells without inflicting injury on the cells.

Moreover, as shown in Fig. 2, the HCV genome could be detected in the negative control - samples 1), 2), 3) and 4), without including any compound, up to 10-fold or 30-fold dilution, however, to the contrary, in the case of the samples added with the compound of Synthetic Example 10, in 1 µM added -samples 5) and 6); and 10 µM added -samples 7) and 8), as well as to the samples added with the compound of Synthetic Example 11, in 1 µM added -samples 9) and 10); and 10 µM added -samples 11) and 12), the HCV genome could not be detected, except for one sample diluted to 10-fold, respectively. On this occasion, yield of total RNA for each of the samples showed little or no alteration. Thus, it was believed that these compounds inhibited the proliferation of HCV within cells without inflicting injury on the cells.

### INDUSTRIAL APPLICABILITY

According to the present invention, excellent therapeutic agents for hepatitis C with little side effects are provided aiming at a medical treatment for hepatitis C caused by HCV.

The present invention was filed Claiming priority of Japanese Patent Application Nos. 2000-394620, 2001-23542, and 2001-105585.

## Claims

1. A therapeutic agent for hepatitis C which comprises a compound selected from a 3'-deoxy-3'-fluorouridine derivative represented by the following general formula (I): 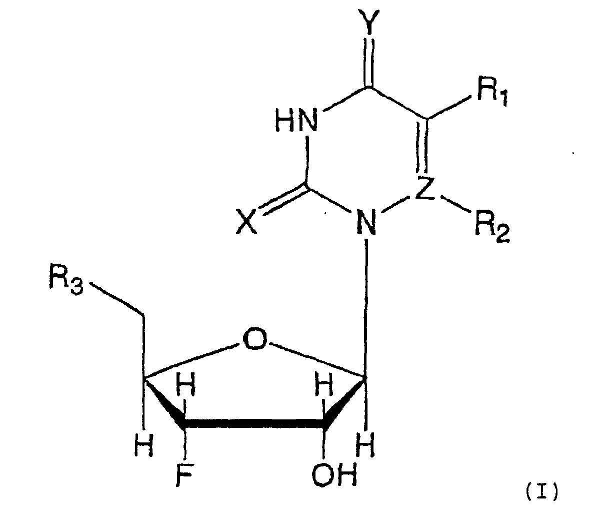 wherein R₁ represents a hydrogen atom, a halogen atom, an alkyl group, an alkyl group substituted by halogen atoms, a hydroxyalkyl group, an aminoalkyl group, an amino group, a formyl group, a cyano group, or an unsubstituted or modified benzyl group. Z represents a carbon atom or a nitrogen atom, and when Z represents a carbon atom, R₂ represents a hydrogen atom, a halogen atom, a formyl group or a cyano group. R₃ represents a hydroxyl group or the following formula: 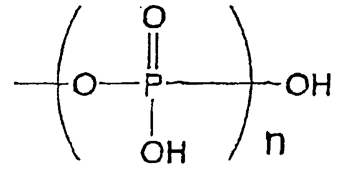 (whereinnrepresents 1, 2 or 3). X and Y each independently represent an oxygen atom or a sulfur atom,
salts thereof and hydrates or solvates of the same as an active ingredient.

2. The therapeutic agent for hepatitis C according to Claim 1 wherein said 3'-deoxy-3'-fluorouridine derivative is any one of 3'-deoxy-3'-fluorouridine,
3'-deoxy-5, 3'-difluorouridine,
3'-deoxy-3'-fluoro-5-methyluridine,
3'-deoxy-3'-fluoro-5-cyanouridine,
3'-deoxy-3'-fluoro-5-trifloromethyluridine,
3'-deoxy-3'-fluoro-5-bromouridine,
3'-deoxy-3'-fluoro-6-azauridine,
3'-deoxy-3'-fluoro-2-thiouridine,
3'-deoxy-3'-fluoro-4-thiouridine, 3'-deoxy-3'-fluorouridine 5'-triphosphate, 3'-deoxy-5,3'-difluorouridine
5'-triphosphate, 3'-deoxy-3'-fluoro-5-methyluridine
5'-triphosphate, 3'-deoxy-3'-fluoro-5-cyanouridine
5'-triphosphate, 3'-deoxy-3'-fluoro-5-trifloromethyluridine 5'-triphosphate, 3'-deoxy-3'-fluoro-5-bromouridine
5'-triphosphate, 3'-deoxy-3'-fluoro-6-azauridine
5'-triphosphate, 3'-deoxy-3'-fluoro-2-thiouridine
5'-triphosphate or 3'-deoxy-3'-fluoro-4-thiouridine
5'-triphosphate.

3. The therapeutic agent for hepatitis C according to Claim 1 wherein said 3'-deoxy-3'-fluorouridine derivative is any one of 3'-deoxy-3'-fluorouridine,
3'-deoxy-5,3'-difluorouridine,
3'-deoxy-3'-fluoro-5-methyluridine,
3'-deoxy-3'-fluorouridine 5'-triphosphate,
3'-deoxy-5,3'-difluorouridine 5'-triphosphate or
3'-deoxy-3'-fluoro-5-methyluridine 5'-triphosphate.

4. The therapeutic agent for hepatitis C according to Claim 1 wherein said 3'-deoxy-3'-fluorouridine derivative is any one of 3'-deoxy-3'-fluorouridine,
3'-deoxy-5,3'-difluorouridine or
3'-deoxy-3'-fluoro-5-methyluridine.

5. The therapeutic agent for hepatitis C according to Claim 1 wherein said 3'-deoxy-3'-fluorouridine derivative is 3'-deoxy-3'-fluorouridine.

6. A compound selected from a
1-(3'-deoxy-3'-fluoro-β-L-ribofuranosyl)uracil derivative represented by the following formula (II): 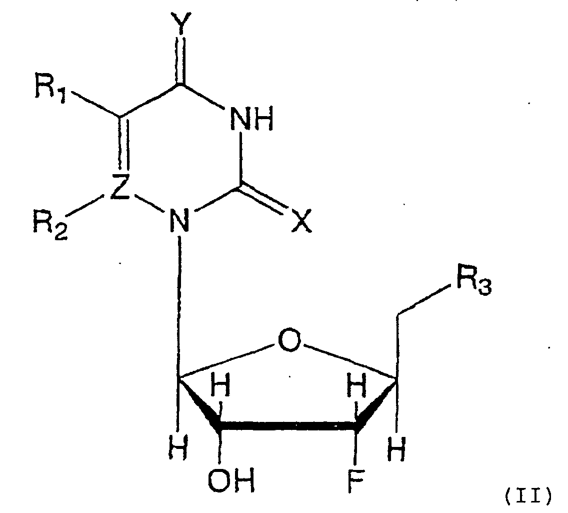 wherein R₁ represents a hydrogen atom, a halogen atom, an alkyl group, an alkyl group substituted by halogen atoms, a hydroxyalkyl group, an aminoalkyl group, an amino group, a formyl group, a cyano group, or an unsubstituted or modified benzyl group. Z represents a carbon atom or a nitrogen atom, and when Z represents a carbon atom, R₂ represents a hydrogen atom, a halogen atom, a formyl group or a cyano group. R₃ represents a hydroxyl group or the following formula: 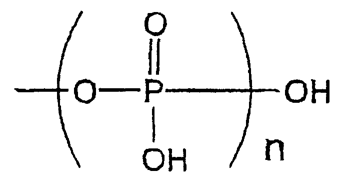 (wherein n represents 1, 2 or 3). X and Y each independently represent an oxygen atom or a sulfur atom,
salts thereof and hydrates or solvates of the same.

7. The compound according to Claim 6 wherein said
1-(3'-deoxy-3'-fluoro-β-L-ribofuranosyl)uracil derivative is any one of 1-(3'-deoxy-3'-fluoro-β-L-ribofuranosyl) uracil,
1-(3'-deoxy-5,3'-difluoro-β-L-ribofuranosyl)uracil,
1-(3'-deoxy-3'-fluoro-5-methyl-β-L-ribofuranosyl)uracil,
1-(3'-deoxy-3'-fluoro-β-L-ribofuranosyl)uracil
5'-triphosphate,
1-(3'-deoxy-5,3'-difluoro-β-L-ribofuranosyl) uracil
5'-triphosphate or
1-(3'-deoxy-3'-fluoro-5-methyl-β-L-ribofuranosyl)uracil
5'-triphosphate.

8. The compound according to Claim 6 wherein said
1-(3'-deoxy-3'-fluoro-β-L-ribofuranosyl)uracil derivative is any one of 1-(3'-deoxy-3'-fluoro-β-L-ribofuranosyl)uracil,
1-(3'-deoxy-5,3'-difluoro-β-L-ribofuranosyl)uracyl,
1-(3'-deoxy-3'-fluoro-5-methyl-β-L-ribofuranosyl)uracil.

9. The compound according to Claim 6 wherein said 1-(3'-deoxy-3'-fluoro-β-L-ribofuranosyl)uracil derivative is 1-(3'-deoxy-3'-fluoro-β-L-ribofuranosyl)uracil.

10. A therapeutic agent for hepatitis C which comprises the compound according to any one of Claims 6 through 9 as an active ingredient.
